# EUROPEAN PATENT SPECIFICATION

(11) **EP 1 304 375 B1**
(45) Date of publication and mention of the grant of the patent: **14.10.2009**
(21) Application number: 01951992.5
(22) Date of filing: 24.07.2001
(51) Int. Cl.: C12N 15/09, C07K 16/06

(54) **METHOD OF PREPARING ANTIBODY BY GENE IMMUNIZATION**
VERFAHREN ZUR HERSTELLUNG VON ANTIKöRPERN DURCH GENIMMUNISIERUNG
PROCEDE DE PREPARATION D'ANTICORPS PAR IMMUNISATION GENIQUE

(30) Priority: 24.07.2000 JP 2000222743; 24.08.2000 JP 2000254407
(43) Date of publication of application: 23.04.2003
(73) Proprietor: Japan Science and Technology Agency, Kawaguchi-shi, Saitama 332-0012 (JP)
(72) Inventor: KATO, Seishi, Sagamihara-shi, Kanagawa 229-0014 (JP); NAGATA, Naoki, Sagamihara-shi, Kanagawa 229-0012 (JP); FUJIMURA, Naoko, Sagamihara-shi, Kanagawa 229-0026 (JP); KOBAYASHI, Midori, Yamato-shi, Kanagawa 242-0006 (JP); ITO, Koichi, Sagamihara-shi, Kanagawa 229-0037 (JP); ISHIZUKA, Yoshiko, Sagamihara-shi, Kanagawa 229-0803 (JP)
(74) Representative: Albutt, Jodie
(86) International application number: PCT/JP2001/006371
(87) International publication number: WO 2002/008416

(56) References cited:
- WAINE G J ET AL: "Genetic immunization of mice with DNA encoding the 23 kDa transmembrane surface protein of Schistosoma japonicum (Sj23) induces antigen-specific immunoglobulin G antibodies" PARASITE IMMUNOLOGY (OXFORD), vol. 21, no. 7, July 1999 (1999-07), pages 377-381, XP002273350 ISSN: 0141-9838
- GUPTA ANUSHREE ET AL: "Identification of novel transmembrane gene sequence and its use for cell-surface targeting of beta subunit of human chorionic gonadotropin" DNA AND CELL BIOLOGY, vol. 17, no. 7, July 1998 (1998-07), pages 573-581, XP001179793 ISSN: 1044-5498
- KATO S. ET AL.: 'Selection of cDNAs encoding putative type II membrane proteins on the cell surface from a human full-length cDNA bank' GENE vol. 228, no. 1-2, 04 March 1999, pages 161 - 167, XP002948593
- LEWIS P.J. ET AL.: 'Altering the cellular location of an antigen expressed by a DNA-based vaccine modulates the immune response' J. VIROL. vol. 73, no. 12, December 1999, pages 10214 - 10223, XP002948594
- BOYLE J.S. ET AL.: 'Influence of cellular location of expressed antigen on the efficacy of DNA vaccination: Cytotoxic T lymphocyte and antibody responses are suboptimal when antigen is cytoplasmic after intramuscular DNA immunization' INT. IMMUNOL. vol. 9, no. 12, December 1997, pages 1897 - 1906, XP002948595
- WAINE G.J. ET AL.: 'DNA immunization by intramuscular injection or gene gun induces specific IgG antibodies against a Schistosoma japonicum 22 kDa antigen, Sj22, when fused to the murine Ig K-chain secretory leader sequence' PARASITE IMMUNOL. vol. 21, no. 1, January 1999, pages 53 - 56, XP002948596
- CHEN Y. ET AL.: 'DNA vaccines encoding full-length or truncated Neu induce protective immunity against Neu-expressing mammary tumors' CANCER RES. vol. 58, no. 9, 01 May 1998, pages 1965 - 1971, XP002948597
- LEWIS P.J. ET AL.: 'Intradermal immunization with a bovine herpesvirus-1 DNA vaccine induces protective immunity in cattle' J. GEN. VIROL. vol. 79, no. PT. 4, April 1998, pages 831 - 839
- XIANG Z.Q. ET AL.: 'Immune response to nucleic acid vaccines to rabies virus' VIROLOGY vol. 209, no. 2, 01 June 1995, pages 569 - 579, XP002948599
- DATABASE BIOSIS [Online] TORRES C.A. ET AL.: 'DNA immunization: effect of secretion of DNA-expressed hemagglutinins on antibody responses', XP002948600 Retrieved from STN Database accession no. 2000:68682 & VACCINE vol. 18, no. 9-10, 10 December 1999, pages 805 - 814
- DATABASE BIOSIS [Online] FOMSGAARD A. ET AL.: 'HIV-1 DNA vaccines', XP002949701 Retrieved from STN Database accession no. 1999:195880 & IMMUNOL. LETT. vol. 65, no. 1-2, January 1999, pages 127 - 131
- DATABASE BIOSIS [Online] DREW D.R. ET AL.: 'Humoral immune responses to DNA vaccines expressing secreted, membrane bound and non-secreted forms of the Tania ovis 45W antigen', XP002949702 Retrieved from STN Database accession no. 2000:280160 & VACCINE vol. 18, no. 23, 22 May 2000, pages 2522 - 2532
- DATABASE BIOSIS [Online] CHENG W.F. ET AL.: 'Enhancement of sindbis virus self-replicating RNA vaccine potency by targeting antigen to endosomal/lysosomal compartments', XP002949703 Retrieved from STN Database accession no. 2001:122889 & HUM. GENE THER. vol. 12, no. 3, 10 February 2001, pages 235 - 252

### SEQUENCE LISTING

<110> Japan Science and Technology Corporation
<120> Method for Producing an Antibody with Genetic Immunization
<130> 01F033PCT
<140> PCT/JP01/06371
   <141> 2001-07-24
<150> JP2000-222743
   <151> 2000-07-24
<150> JP2000-254407
   <151> 2000-08-24
<160> 13
<170> PatentIn Ver. 2.1
<210> 1
   <211> 697
   <212> DNA
   <213> Homo sapiens
<220>
   <221> CDS
   <222> (151).. (600)
<400> 1
   tatacctcta gtttggagct gtgctgtaaa aacaagagta acatttttat attaaagtta 60
   aataaagtta caactttgaa gagagtttct gcaagacatg acacaaagct gctagcagaa 120
   aatcaaaacg ctgattaaaa gaagcacggt atg atg acc aaa cat aaa aag tgt  174
                                    Met Met Thr Lys His Lys Lys Cys
                                      1               5
   ttt ata att gtt ggt gtt tta ata aca act aat att att act ctg ata   222
   Phe Ile Ile Val Gly Val Leu Ile Thr Thr Asn Ile Ile Thr Leu Ile
        10                  15                  20
   gtt aaa cta act cga gat tct cag agt tta tgc ccc tat gat tgg att   270
   Val Lys Leu Thr Arg Asp Ser Gln Ser Leu Cys Pro Tyr Asp Trp Ile
   25 30 35 40
   ggt ttc caa aac aaa tgc tat tat ttc tct aaa gaa gaa gga gat tgg 318
   Gly Phe Gln Asn Lys Cys Tyr Tyr Phe Ser Lys Glu Glu Gly Asp Trp
   45 50 55
   aat tca agt aaa tac aac tgt tcc act caa cat gcc gac cta act ata 366
   Asn Ser Ser Lys Tyr Asn Cys Ser Thr Gln His Ala Asp Leu Thr Ile
   60 65 70
   att gac aac ata gaa gaa atg aat ttt ctt agg cgg tat aaa tgc agt 414
   Ile Asp Asn Ile Glu Glu Met Asn Phe Leu Arg Arg Tyr Lys Cys Ser
   75 80 85
   tct gat cac tgg att gga ctg aag atg gca aaa aat cga aca gga caa 462
   Ser Asp His Trp Ile Gly Leu Lys Met Ala Lys Asn Arg Thr Gly Gln
   90 95 100
   tgg gta gat gga gct aca ttt acc aaa tcg ttt ggc atg aga ggg agt 510
   Trp Val Asp Gly Ala Thr Phe Thr Lys Ser Phe Gly Met Arg Gly Ser
   105 110 115 120
   gaa gga tgt gcc tac ctc agc gat gat ggt gca gca aca gct aga tgt 558
   Glu Gly Cys Ala Tyr Leu Ser Asp Asp Gly Ala Ala Thr Ala Arg Cys
   125 130 135
   tac acc gaa aga aaa tgg att tgc agg aaa aga ata cac taa 600
   Tyr Thr Glu Arg Lys Trp Ile Cys Arg Lys Arg Ile His
   140 145 150
   gttaatgtct aagataatgg ggaaaataga aaataacatt attaagtgta aaaccagcaa 660
   agtacttttt taattaaaca aagttcgagt tttgtac 697
<210> 2
   <211> 149
   <212> PRT
   <213> Homo sapiens
<400> 2
   Met Met Thr Lys His Lys Lys Cys Phe Ile Ile Val Gly Val Leu Ile
   1 5 10 15
   Thr Thr Asn Ile Ile Thr Leu Ile Val Lys Leu Thr Arg Asp Ser Gln
   20 25 30
   Ser Leu Cys Pro Tyr Asp Trp Ile Gly Phe Gln Asn Lys Cys Tyr Tyr
           35                  40                  45
   Phe Ser Lys Glu Glu Gly Asp Trp Asn Ser Ser Lys Tyr Asn Cys Ser
       50                  55                  60
   Thr Gln His Ala Asp Leu Thr Ile Ile Asp Asn Ile Glu Glu Met Asn
   65                  70                  75                  80
   Phe Leu Arg Arg Tyr Lys Cys Ser Ser Asp His Trp Ile Gly Leu Lys
                   85                  90                  95
   Met Ala Lys Asn Arg Thr Gly Gln Trp Val Asp Gly Ala Thr Phe Thr
               100                 105                 110
   Lys Ser Phe Gly Met Arg Gly Ser Glu Gly Cys Ala Tyr Leu Ser Asp
           115                 120                 125
   Asp Gly Ala Ala Thr Ala Arg Cys Tyr Thr Glu Arg Lys Trp Ile Cys
       130                 135                 140
   Arg Lys Arg Ile His
   145
<210> 3
   <211> 548
   <212> DNA
   <213> Homo sapiens
<220>
   <221> CDS
   <222> (30).. (503)
<400> 3
   cttattgctg gcggcctgag gagcccatc atg gcg acg ccc cct aag cgg cgg    53
                                   Met Ala Thr Pro Pro Lys Arg Arg
                                   1               5
   gcg gtg gag gcc acg ggg gag aaa gtg ctg cgc tac gag acc ttc atc   101
   Ala Val Glu Ala Thr Gly Glu Lys Val Leu Arg Tyr Glu Thr Phe Ile
       10                  15                  20
   agt gac gtg ctg cag cgg gac ttg cga aag gtg ctg gac cat cga gac   149
   Ser Asp Val Leu Gln Arg Asp Leu Arg Lys Val Leu Asp His Arg Asp
   25                  30                  35                  40
   aag gta tat gag cag ctg gcc aaa tac ctt caa ctg aga aat gtc att   197
   Lys Val Tyr Glu Gln Leu Ala Lys Tyr Leu Gln Leu Arg Asn Val Ile
                   45                  50                  55
   gag cga ctc cag gaa gct aag cac tcg gag tta tat atg cag gtg gat   245
   Glu Arg Leu Gln Glu Ala Lys His Ser Glu Leu Tyr Met Gln Val Asp
               60                  65                  70
   ttg ggc tgt aac ttc ttc gtt gac aca gtg gtc cca gat act tca cgc   293
   Leu Gly Cys Asn Phe Phe Val Asp Thr Val Val Pro Asp Thr Ser Arg
           75                  80                  85
   atc tat gtg gcc ctg gga tat ggt ttt ttc ctg gag ttg aca ctg gca   341
   Ile Tyr Val Ala Leu Gly Tyr Gly Phe Phe Leu Glu Leu Thr Leu Ala
       90                  95                  100
   gaa gct ctc aag ttc att gat cgt aag agc tct ctc ctc aca gag ctc   389
   Glu Ala Leu Lys Phe Ile Asp Arg Lys Ser Ser Leu Leu Thr Glu Leu
   105                 110                 115                 120
   agc aac agc ctc acc aag gac tcc atg aat atc aaa gcc cat atc cac   437
   Ser Asn Ser Leu Thr Lys Asp Ser Met Asn Ile Lys Ala His Ile His
                   125                 130                 135
   atg ttg cta gag ggg ctt aga gaa cta caa ggc ctg cag aat ttc cca   485
   Met Leu Leu Glu Gly Leu Arg Glu Leu Gln Gly Leu Gln Asn Phe Pro
               140                 145                 150
   gag aag cct cac cat tga cttcttcccc ccatcctcag acattaaaga          533
   Glu Lys Pro His His
           155
   gcctgaatgc ctttg                                                  548
<210> 4
   <211> 157
   <212> PRT
   <213> Homo sapiens
<400> 4
   Met Ala Thr Pro Pro Lys Arg Arg Ala Val Glu Ala Thr Gly Glu Lys
   1               5                   10                  15
   Val Leu Arg Tyr Glu Thr Phe Ile Ser Asp Val Leu Gln Arg Asp Leu
               20                  25                  30
   Arg Lys Val Leu Asp His Arg Asp Lys Val Tyr Glu Gln Leu Ala Lys
           35                  40                  45
   Tyr Leu Gln Leu Arg Asn Val Ile Glu Arg Leu Gln Glu Ala Lys His
       50                  55                  60
   Ser Glu Leu Tyr Met Gln Val Asp Leu Gly Cys Asn Phe Phe Val Asp
   65 70 75 80
   Thr Val Val Pro Asp Thr Ser Arg Ile Tyr Val Ala Leu Gly Tyr Gly
   85 90 95
   Phe Phe Leu Glu Leu Thr Leu Ala Glu Ala Leu Lys Phe Ile Asp Arg
   100 105 110
   Lys Ser Ser Leu Leu Thr Glu Leu Ser Asn Ser Leu Thr Lys Asp Ser
   115 120 125
   Met Asn Ile Lys Ala His Ile His Met Leu Leu Glu Gly Leu Arg Glu
   130 135 140
   Leu Gln Gly Leu Gln Asn Phe Pro Glu Lys Pro His His
   145 150 155
<210> 5
   <211> 30
   <212> DNA
   <213> Artificial Sequence
<220>
   <223> Artificial Sequence: Synthesized oligonucleotide
<400> 5
   cccgatatct catggcgacg ccccctaagc 30
<210> 6
   <211> 30
   <212> DNA
   <213> Artificial Sequence
<220>
   <223> Artificial Sequence: Synthesized oligonucleotide
<400> 6
   cccgatatct caatggtgag gcttctctgg 30
<210> 7
   <211> 28
   <212> DNA
   <213> Artificial Sequence
<220>
   <223> Artificial Sequence: Synthesized oligonucleotide
<400> 7
   cccgaattca tggcgacgcc ccctaagc 28
<210> 8
   <211> 32
   <212> DNA
   <213> Artificial Sequence
<220>
   <223> Artificial Sequence: Synthesized oligonucleotide
<400> 8
   cccgtcgacg catggtgagg cttctctggg aa 32
<210> 9
   <211> 1643
   <212> DNA
   <213> Homo sapiens
<220>
   <221> CDS
   <222> (25).. (915)
<400> 9
   aacatctggg gacagcggga aaac atg agt gac tcc aag gaa cca agg gtg     51
                              Met Ser Asp Ser Lys Glu Pro Arg Val
                              1               5
   cag cag ctg ggc ctc ctg ggg tgt ctt ggc cat ggc gcc ctg gtg ctg    99
   Gln Gln Leu Gly Leu Leu Gly Cys Leu Gly His Gly Ala Leu Val Leu
   10                  15                  20                  25
   caa ctc ctc tcc ttc atg ctc ttg gct ggg gtc ctg gtg gcc atc ctt    147
   Gln Leu Leu Ser Phe Met Leu Leu Ala Gly Val Leu Val Ala Ile Leu
                   30                  35                  40
   gtc caa gtg tcc aag gtc ccc agc tcc cta agt cag gaa caa tcc gag    195
   Val Gln Val Ser Lys Val Pro Ser Ser Leu Ser Gln Glu Gln Ser Glu
               45                  50                  55
   caa gac gca atc tac cag aac ctg acc cag ctt aaa gct gca gtg ggt    243
   Gln Asp Ala Ile Tyr Gln Asn Leu Thr Gln Leu Lys Ala Ala Val Gly
           60                  65                  70
   gag ctc tca gag aaa tcc aag ctg cag gag atc tac cag gag ctg acc   291
   Glu Leu Ser Glu Lys Ser Lys Leu Gln Glu Ile Tyr Gln Glu Leu Thr
        75                  80                  85
   cag ctg aag gct gca gtg ggt gag ttg cca gag aaa tcc aag ctg cag   339
   Gln Leu Lys Ala Ala Val Gly Glu Leu Pro Glu Lys Ser Lys Leu Gln
    90                  95                 100                 105
   gag atc tac cag gag ctg acc cgg ctg aag gct gca gtg ggt gag ttg   387
   Glu Ile Tyr Gln Glu Leu Thr Arg Leu Lys Ala Ala Val Gly Glu Leu
                   110                 115                 120
   cca gag aaa tcc aag ctg cag gag atc tac cag gag ctg acc cgg ctg   435
   Pro Glu Lys Ser Lys Leu Gln Glu Ile Tyr Gln Glu Leu Thr Arg Leu
               125                 130                 135
   aag gct gca gtg ggt gag ttg cca gag aaa tcc aag ctg cag gag atc   483
   Lys Ala Ala Val Gly Glu Leu Pro Glu Lys Ser Lys Leu Gln Glu Ile
           140                 145                 150
   tac cag gag ctg acc cgg ctg aag gct gca gtg ggt gag ttg cca gag   531
   Tyr Gln Glu Leu Thr Arg Leu Lys Ala Ala Val Gly Glu Leu Pro Glu
       155                 160                 165
   aaa tcc aag ctg cag gag atc tac cag gag ctg acg gag ctg aag gct   579
   Lys Ser Lys Leu Gln Glu Ile Tyr Gln Glu Leu Thr Glu Leu Lys Ala
   170                 175                 180                 185
   gca gtg ggt gag ttg cca gag aaa tcc aag ctg cag gag atc tac cag   627
   Ala Val Gly Glu Leu Pro Glu Lys Ser Lys Leu Gln Glu Ile Tyr Gln
                   190                 195                 200
   gag ctg acc cag ctg aag gct gca gtg ggt gag ttg cca gac cag tcc   675
   Glu Leu Thr Gln Leu Lys Ala Ala Val Gly Glu Leu Pro Asp Gln Ser
               205                 210                 215
   aag cag cag caa atc tat caa gaa ctg acc gat ttg aag act gca ttt   723
   Lys Gln Gln Gln Ile Tyr Gln Glu Leu Thr Asp Leu Lys Thr Ala Phe
           220                 225                 230
   gaa cgc ctg tgc cgc cac tgt ccc aag gac tgg aca ttc ttc caa gga   771
   Glu Arg Leu Cys Arg His Cys Pro Lys Asp Trp Thr Phe Phe Gln Gly
       235                 240                 245
   aac tgt tac ttc atg tct aac tcc cag cgg aac tgg cac gac tcc gtc 819
   Asn Cys Tyr Phe Met Ser Asn Ser Gln Arg Asn Trp His Asp Ser Val
   250 255 260 265
   acc gcc tgc cag gaa gtg agg gcc cag ctc gtc gta atc aaa act gct 867
   Thr Ala Cys Gln Glu Val Arg Ala Gln Leu Val Val Ile Lys Thr Ala
   270 275 280
   gag gag cag ctt cca gcg gta ctg gaa cag tgg aga acc caa caa tag 915
   Glu Glu Gln Leu Pro Ala Val Leu Glu Gln Trp Arg Thr Gln Gln
   285 290 295
   cgggaatgaa gactgtgcgg aatttagtgg cagtggctgg aacgacaatc gatgtgacgt 975
   tgacaattac tggatctgca aaaagcccgc agcctgcttc agagacgaat agttgtttcc 1035
   ctgctagcct cagcctccat tgtggtatag cagaacttca cccacttgta agccagcgct 1095
   tcttctctcc atccttggac cttcacaaat gccctgagac ggttctctgt tcgatttttc 1155
   atcccctatg aacctgggtc ttattctgtc cttctgatgc ctccaagttt ccctggtgta 1215
   gagcttgtgt tcttggccca tccttggagc tttataagtg acctgagtgg gatgcattta 1275
   gggggcgggc ttggtatgtt gtatgaatcc actctctgtt ccttttggag attagactat 1335
   ttggattcat gtgtagctgc cctgtcccct ggggctttat ctcatccatg caaactacca 1395
   tctgctcaac ttccagctac accccgtgca cccttttgac tggggacttg ctggttgaag 1455
   gagctcatct tgcaggctgg aagcaccagg gaattaattc ccccagtcaa ccaatggcat 1515
   ccagagaggg catggaggct ccatacaacc tcttccaccc ccacatcttt ctttgtccta 1575
   tacatgtctt ccatttggct gtttctgagt tgtagccttt ataataaagt ggtaaatgtt 1635
   gtaactgc 1643
<210> 10
   <211> 2186
   <212> DNA
   <213> Homo sapiens
<220>
   <221> CDS
   <222> (118).. (1236)
<400> 10
   actctttctt cggctcgcga gctgagagga gcaggtagag gggcagaggc gggactgtcg 60
   tctgggggag ccgcccagga ggctcctcag gccgacccca gaccctggct ggccagg    117
   atg aag tat ctc cgg cac cgg cgg ccc aat gcc acc ctc att ctg gcc   165
   Met Lys Tyr Leu Arg His Arg Arg Pro Asn Ala Thr Leu Ile Leu Ala
    1               5                  10                  15
   atc ggc gct ttc acc ctc ctc ctc ttc agt ctg cta gtg tca cca ccc   213
   Ile Gly Ala Phe Thr Leu Leu Leu Phe Ser Leu Leu Val Ser Pro Pro
               20                  25                  30
   acc tgc aag gtc cag gag cag cca ccg gcg atc ccc gag gcc ctg gcc   261
   Thr Cys Lys Val Gln Glu Gln Pro Pro Ala Ile Pro Glu Ala Leu Ala
           35                  40                  45
   tgg ccc act cca ccc acc cgc cca gcc ccg gcc ccg tgc cat gcc aac   309
   Trp Pro Thr Pro Pro Thr Arg Pro Ala Pro Ala Pro Cys His Ala Asn
       50                  55                  60
   acc tct atg gtc acc cac ccg gac ttc gcc acg cag ccg cag cac gtt   357
   Thr Ser Met Val Thr His Pro Asp Phe Ala Thr Gln Pro Gln His Val
   65                  70                  75                  80
   cag aac ttc ctc ctg tac aga cac tgc cgc cac ttt ccc ctg ctg cag   405
   Gln Asn Phe Leu Leu Tyr Arg His Cys Arg His Phe Pro Leu Leu Gln
                   85                  90                  95
   gac gtg ccc ccc tct aag tgc gcg cag ccg gtc ttc ctg ctg ctg gtg   453
   Asp Val Pro Pro Ser Lys Cys Ala Gln Pro Val Phe Leu Leu Leu Val
               100                 105                 110
   atc aag tcc tcc cct agc aac tat gtg cgc cgc gag ctg ctg cgg cgc   501
   Ile Lys Ser Ser Pro Ser Asn Tyr Val Arg Arg Glu Leu Leu Arg Arg
           115                 120                 125
   acg tgg ggc cgc gag cgc aag gta cgg ggt ttg cag ctg cgc ctc ctc   549
   Thr Trp Gly Arg Glu Arg Lys Val Arg Gly Leu Gln Leu Arg Leu Leu
       130                 135                 140
   ttc ctg gtg ggc aca gcc tcc aac ccg cac gag gcc cgc aag gtc aac   597
   Phe Leu Val Gly Thr Ala Ser Asn Pro His Glu Ala Arg Lys Val Asn
   145 150 155 160
   cgg ctg ctg gag ctg gag gca cag act cac gga gac atc ctg cag tgg 645
   Arg Leu Leu Glu Leu Glu Ala Gln Thr His Gly Asp Ile Leu Gln Trp
   165 170 175
   gac ttc cac gac tcc ttc ttc aac ctc acg ctc aag cag gtc ctg ttc 693
   Asp Phe His Asp Ser Phe Phe Asn Leu Thr Leu Lys Gln Val Leu Phe
   180 185 190
   tta cag tgg cag gag aca agg tgc gcc aac gcc agc ttc gtg ctc aac 741
   Leu Gln Trp Gln Glu Thr Arg Cys Ala Asn Ala Ser Phe Val Leu Asn
   195 200 205
   ggg gat gat gac gtc ttt gca cac aca gac aac atg gtc ttc tac ctg 789
   Gly Asp Asp Asp Val Phe Ala His Thr Asp Asn Met Val Phe Tyr Leu
   210 215 220
   cag gac cat gac cct ggc cgc cac ctc ttc gtg ggg caa ctg atc caa 837
   Gln Asp His Asp Pro Gly Arg His Leu Phe Val Gly Gln Leu Ile Gln
   225 230 235 240
   aac gtg ggc ccc atc cgg gct ttt tgg agc aag tac tat gtg cca gag 885
   Asn Val Gly Pro Ile Arg Ala Phe Trp Ser Lys Tyr Tyr Val Pro Glu
   245 250 255
   gtg gtg act cag aat gag cgg tac cca ccc tat tgt ggg ggt ggt ggc 933
   Val Val Thr Gln Asn Glu Arg Tyr Pro Pro Tyr Cys Gly Gly Gly Gly
   260 265 270
   ttc ttg ctg tcc cgc ttc acg gcc gct gcc ctg cgc cgt gct gcc cat 981
   Phe Leu Leu Ser Arg Phe Thr Ala Ala Ala Leu Arg Arg Ala Ala His
   275 280 285
   gtc ttg gac atc ttc ccc att gat gat gtc ttc ctg ggt atg tgt ctg 1029
   Val Leu Asp Ile Phe Pro Ile Asp Asp Val Phe Leu Gly Met Cys Leu
   290 295 300
   gag ctt gag gga ctg aag cct gcc tcc cac agc ggc atc cgc acg tct 1077
   Glu Leu Glu Gly Leu Lys Pro Ala Ser His Ser Gly Ile Arg Thr Ser
   305 310 315 320
   ggc gtg cgg gct cca tcg caa cac ctg tcc tcc ttt gac ccc tgc ttc 1125
   Gly Val Arg Ala Pro Ser Gln His Leu Ser Ser Phe Asp Pro Cys Phe
                   325                 330                 335
   tac cga gac ctg ctg ctg gtg cac cgc ttc cta cct tat gag atg ctg   1173
   Tyr Arg Asp Leu Leu Leu Val His Arg Phe Leu Pro Tyr Glu Met Leu
               340                 345                 350
   ctc atg tgg gat gcg ctg aac cag ccc aac ctc acc tgc ggc aat cag   1221
   Leu Met Trp Asp Ala Leu Asn Gln Pro Asn Leu Thr Cys Gly Asn Gln
           355                 360                 365
   aca cag atc tac tga gtcagcatca gggtccccag cctctgggct cctgtttcca   1276
   Thr Gln Ile Tyr
       370
   taggaagggg cgacaccttc ctcccaggaa gctgagacct ttgtggtctg agcataaggg 1336
   agtgccaggg aaggtttgag gtttgatgag tgaatattct ggctggcgaa ctcctacaca 1396
   tccttcaaaa cccacctggt actgttccag catcttccct ggatggctgg aggaactcca 1456
   gaaaatatcc atcttctttt tgtggctgct aatggcagaa gtgcctgtgc tagagttcca 1516
   actgtggatg catccgtccc gtttgagtca aagtcttact tccctgctct cacctactca 1576
   cagacgggat gctaagcagt gcacctgcag tggtttaatg gcagataagc tccgtctgca 1636
   gttccaggcc agccagaaac tcctgtgtcc acatagagct gacgtgagaa atatctttca 1696
   gcccaggaga gaggggtcct gatcttaacc ctttcctggg tctcagacaa ctcagaaggt 1756
   tggggggata ccagagaggt ggtggaatag gaccgccccc tccttacttg tgggatcaaa 1816
   tgctgtaatg gtggaggtgt gggcagagga gggaggcaag tgtcctttga aagttgtgag 1876
   agctcagagt ttctggggtc ctcattagga gcccccatcc ctgtgttccc caagaattca 1936
   gagaacagca ctggggctgg aatgatcttt aatgggccca aggccaacag gcatatgcct 1996
   cactactgcc tggagaaggg agagattcag gtcctccagc agcctccctc acccagtatg 2056
   ttttacagat tacgggggga ccgggtgagc cagtgacccc ctgcagcccc cagcttcagg 2116
   cctcagtgtc tgccagtcaa gcttcacagg cattgtgatg gggcagcctt ggggaatata 2176
   aaattttgtg                                                        2186
<210> 11
   <211> 814
   <212> DNA
   <213> Homo sapiens
<220>
   <221> CDS
   <222> (145).. (693)
<400> 11
   agaatcccgg acagccctgc tccctgcagc caggtgtagt ttcgggagcc actggggcca 60
   aagtgagagt ccagcggtct tccagcgctt gggccacggc ggcggccctg ggagcagagg 120
   tggagcgacc ccattacgct aaag atg aaa ggc tgg ggt tgg ctg gcc ctg   171
                              Met Lys Gly Trp Gly Trp Leu Ala Leu
                              1               5
   ctt ctg ggg gcc ctg ctg gga acc gcc tgg gct cgg agg agc cag gat  219
   Leu Leu Gly Ala Leu Leu Gly Thr Ala Trp Ala Arg Arg Ser Gln Asp
   10                  15                  20                  25
   ctc cac tgt gga gca tgc agg gct ctg gtg gat gaa cta gaa tgg gaa  267
   Leu His Cys Gly Ala Cys Arg Ala Leu Val Asp Glu Leu Glu Trp Glu
                   30                  35                  40
   att gcc cag gtg gac ccc aag aag acc att cag atg gga tct ttc cgg  315
   Ile Ala Gln Val Asp Pro Lys Lys Thr Ile Gln Met Gly Ser Phe Arg
               45                  50                  55
   atc aat cca gat ggc agc cag tca gtg gtg gag gtg cct tat gcc cgc  363
   Ile Asn Pro Asp Gly Ser Gln Ser Val Val Glu Val Pro Tyr Ala Arg
           60                  65                  70
   tca gag gcc cac ctc aca gag ctg ctg gag gag ata tgt gac cgg atg  411
   Ser Glu Ala His Leu Thr Glu Leu Leu Glu Glu Ile Cys Asp Arg Met
       75                  80                  85
   aag gag tat ggg gaa cag att gat cct tcc acc cat cgc aag aac tac  459
   Lys Glu Tyr Gly Glu Gln Ile Asp Pro Ser Thr His Arg Lys Asn Tyr
   90                  95                  100                 105
   gta cgt gta gtg ggc cgg aat gga gaa tcc agt gaa ctg gac cta caa  507
   Val Arg Val Val Gly Arg Asn Gly Glu Ser Ser Glu Leu Asp Leu Gln
   110 115 120
   ggc atc cga atc gac tca gat att agc ggc acc ctc aag ttt gcg tgt 555
   Gly Ile Arg Ile Asp Ser Asp Ile Ser Gly Thr Leu Lys Phe Ala Cys
   125 130 135
   gag agc att gtg gag gaa tac gag gat gaa ctc att gaa ttc ttt tcc 603
   Glu Ser Ile Val Glu Glu Tyr Glu Asp Glu Leu Ile Glu Phe Phe Ser
   140 145 150
   cga gag gct gac aat gtt aaa gac aaa ctt tgc agt aag cga aca gat 651
   Arg Glu Ala Asp Asn Val Lys Asp Lys Leu Cys Ser Lys Arg Thr Asp
   155 160 165
   ctt tgt gac cat gcc ctg cac ata tcg cat gat gag cta tga 693
   Leu Cys Asp His Ala Leu His Ile Ser His Asp Glu Leu
   170 175 180
   accactggag cagcccacac tggcttgatg gatcaccccc aggaggggaa aatggtggca 753
   atgcctttta tatattatgt ttttactgaa attaactgaa aaaatatgaa accaaaagta 813
   c 814
<210> 12
   <211> 695
   <212> DNA
   <213> Homo sapiens
<220>
   <221> CDS
   <222> (73).. (564)
<400> 12
   aagatttcag ctgcgggacg gtcaggggag acctccaggc gcagggaagg acggccaggg 60
   tgacacggaa gc atg cga cgg ctg ctg atc cct ctg gcc ctg tgg ctg ggc 111
   Met Arg Arg Leu Leu Ile Pro Leu Ala Leu Trp Leu Gly
   1 5 10
   gcg gtg ggc gtg ggc gtc gcc gag ctc acg gaa gcc cag cgc cgg ggc 159
   Ala Val Gly Val Gly Val Ala Glu Leu Thr Glu Ala Gln Arg Arg Gly
   15 20 25
   ctg cag gtg gcc ctg gag gaa ttt cac aag cac ccg ccc gtg cag tgg    207
   Leu Gln Val Ala Leu Glu Glu Phe His Lys His Pro Pro Val Gln Trp
    30                  35                  40                  45
   gcc ttc cag gag acc agt gtg gag agc gcc gtg gac acg ccc ttc cca    255
   Ala Phe Gln Glu Thr Ser Val Glu Ser Ala Val Asp Thr Pro Phe Pro
                    50                  55                  60
   gct gga ata ttt gtg agg ctg gaa ttt aag ctg cag cag aca agc tgc    303
   Ala Gly Ile Phe Val Arg Leu Glu Phe Lys Leu Gln Gln Thr Ser Cys
                65                  70                  75
   cgg aag agg gac tgg aag aaa ccc gag tgc aaa gtc agg ccc aat ggg    351
   Arg Lys Arg Asp Trp Lys Lys Pro Glu Cys Lys Val Arg Pro Asn Gly
            80                  85                  90
   agg aaa cgg aaa tgc ctg gcc tgc atc aaa ctg ggc tct gag gac aaa    399
   Arg Lys Arg Lys Cys Leu Ala Cys Ile Lys Leu Gly Ser Glu Asp Lys
        95                 100                 105
   gtt ctg ggc cgg ttg gtc cac tgc ccc ata gag acc caa gtt ctg cgg    447
   Val Leu Gly Arg Leu Val His Cys Pro Ile Glu Thr Gln Val Leu Arg
   110                 115                 120                 125
   gag gct gag gag cac cag gag acc cag tgc ctc agg gtg cag cgg gct    495
   Glu Ala Glu Glu His Gln Glu Thr Gln Cys Leu Arg Val Gln Arg Ala
                   130                 135                 140
   ggt gag gac ccc cac agc ttc tac ttc cct gga cag ttc gcc ttc tcc    543
   Gly Glu Asp Pro His Ser Phe Tyr Phe Pro Gly Gln Phe Ala Phe Ser
               145                 150                 155
   aag gcc ctg ccc cgc agc taa gccagcactg agctgcgtgg tgcctccagg       594
   Lys Ala Leu Pro Arg Ser
           160
   accgctgccg gtggtaacca gtggaagacc ccagccccca gggagaggac cccgttctat 654
   ccccagccat gataataaag ctgctctccc agctgcctct c                      695
<210> 13
   <211> 1451
   <212> DNA
   <213> Homo sapiens
<220>
   <221> CDS
   <222> (105).. (1436)
<400> 13
actgcctgga aacgggctgg gcctgcctcg gacgccgccg gtgtcgcgga ttctctttcc 60
   gcccgctcca tggcggtgga tgcctgactg gaagcccgag tggg atg cgg ctg acg  116
   cgg aag cgg ctc tgc tcg ttt ctt atc gcc ctg tac tgc cta ttc tcc  164
   ctc tac gct gcc tac cac gtc ttc ttc ggg cgc cgc cgc cag gcg ccg  212
   gcc ggg tcc ccg cgg ggc ctc agg aag ggg gcg gcc ccc gcg cgg gag  260
   aga cgc ggc cga gaa cag tcc act ttg gaa agt gaa gaa tgg aat cct  308
   tgg gaa gga gat gaa aaa aat gag caa caa cac aga ttt aaa act agc  356
   ctt caa ata tta gat aaa tcc acg aaa gga aaa aca gat ctc agt gta  404
   caa atc tgg ggc aaa gct gcc att ggc ttg tat ctc tgg gag cat att  452
   ttt gaa ggc tta ctt gat ccc agc gat gtg act gct caa tgg aga gaa  500
   gga aag tca atc gta gga aga aca cag tac agc ttc atc act ggt cca   548
   gct gta ata cca ggg tac ttc tcc gtt gat gtg aat aat gtg gta ctc   596
   att tta aat gga aga gaa aaa gca aag atc ttt tat gcc acc cag tgg   644
   tta ctt tat gca caa aat tta gtg caa att caa aaa ctc cag cat ctt   692
   gct gtt gtt ttg ctc gga aat gaa cat tgt gat aat gag tgg ata aac   740
   cca ttc ctc aaa aga aat gga ggc ttc gtg gag ctg ctt ttc ata ata   788
   tat gac agc ccc tgg att aat gac gtg gat gtt ttt cag tgg cct tta   836
   gga gta gca aca tac agg aat ttt cct gtg gtg gag gca agt tgg tca   884
   atg ctg cat gat gag agg cca tat tta tgt aat ttc tta gga acg att   932
   tat gaa aat tca tcc aga cag gca cta atg aac att ttg aaa aaa gat   980
   ggg aac gat aag ctt tgt tgg gtt tca gca aga gaa cac tgg cag cct   1028
   cag gaa aca aat gaa agt ctt aag aat tac caa gat gcc ttg ctt cag   1076
   agt gat ctc aca ttg tgc ccg gtc gga gta aac aca gaa tgc tat cga 1124
   atc tat gag gct tgc tcc tat ggc tcc att cct gtg gtg gaa gac gtg 1172
   atg aca gct ggc aac tgt ggg aat aca tct gtg cac cac ggt gct cct 1220
   ctg cag tta ctc aag tcc atg ggt gct ccc ttt atc ttt atc aag aac 1268
   tgg aag gaa ctc cct gct gtt tta gaa aaa gag aaa act ata att tta 1316
   caa gaa aaa att gaa aga aga aaa atg tta ctt cag tgg tat cag cac 1364
   ttc aag aca gag ctt aaa atg aaa ttt act aat att tta gaa agc tca 1412
   ttt tta atg aat aat aaa agt taa ttatcttttt gagct 1451

## Description

### Technical Field

The invention of the present application relates to a method for producing an antibody by gene immunization. More specifically, the invention relates to a method of enabling easy production of an antibody useful as drugs, diagnostic agents, reagents for the research, and etc., and to an expression vector used in this method.

### Background Art

An antibody has widely been utilized as reagents for the research for the purpose of detection, purification, elimination, inhibition of a protein or the like, because it has property of recognizing specific protein and binding thereto. Recently, it has widely been used not only as reagents for the research but also as drugs or diagnostic agents.

In producing antibodies, it has so far been general to use a method that a large amount of protein as an antigen is purified and injected to an animal or animals such as rabbits or mice to collect antibodies generated in sera. It required, however, much time and a great deal of labor to obtain a large amount of a purified antigenic protein. It is desired to provide a more convenient method for producing antibodies, accordingly.

Recently, it was reported that when a gene coding for an influenza virus nucleoprotein is integrated into an expression vector and intramuscularly injected directly as DNA to mice, then virus proteins are produced in the murine bodies and additionally the antibody against these proteins are generated in the sera. (Ulmer et al., Science 259: 1745-1749, 1993; Ginsbert et al., "Vaccines 93"). As a result, this expression vector received much attention as a new type of vaccine, that is, DNA vaccine, since mice have acquired immunity to virus. Thus, it has been designated as gene immunization that an expression vector for an antigenic protein is inoculated directly to an animal to generate immunity. In using gene immunization, however, in some cases, the titer of the generated antibody is very low or no antibody is generated depending on the kind of the antigen used.

It was reported as an example of gene immunization that ovalbumin was fused in the downstream of transmembrane domain of transferrin receptor to form a membrane type and it was injected intramuscularly or subcutaneously to mice in order to investigate an effect of the expression site of antigenic protein on the efficacy of gene immunization. The titer of the antibodies generated, however, rather decreased since the protein was converted into a membrane type. (Boyle et al., Int. Immunol. 9: 1897-1906, 1997).

The purpose of the invention of present application is to provide a method for producing antibodies to proteins, which it was difficult to produce in so far known gene immunization methods.

Additionally, the purpose of the application is to provide an expression vector used in the above-mentioned method for producing an antibody.

### Disclosure of the Invention

The present application, as the invention for solving the above-described problems, provides a method for producing an antibody which comprises inoculating an expression vector expressing a fusion protein to an animal, isolating an antibody against an antigenic protein from the animal and purifying the antibody, wherein the fusion protein is an antigenic protein fused with the C-terminal side of a transmembrane domain of which the N-terminal side is located in the cell and the C-terminal side is out of the cell.

In a preferred embodiment in this method for producing an antibody, the transmembrane domain is a polypeptide having at least the amino acid sequence from 1st to 26th of SEQ ID NO. 2.

The application also provides the use of an expression vector expressing a fusion protein in which an antigenic protein is fused with the C-terminal side of transmembrane domain of which the N-terminal side is located in the cell and the C-terminal side is out of the cell for the production of an antibody.

In a preferred embodiment of this expression vector, the transmembrane domain is a polypeptide having at least the amino acid sequence from 1st to 26th of SEQ ID NO. 2.

### Brief Description of the Drawings

Fig. 1 shows the structure of urokinase fusion gene.
Fig. 2 is an example illustrative of determination of antibody titer by ELISA when gene immunization has been carried out with a urokinase expression vector.
Fig. 3 is an example illustrative of determination of antibody titer by ELISA when gene immunization has been carried out with a nuclear protein HP 10496 expression vector.
Fig. 4 shows the respective N-terminal amino acid sequences of fusion proteins comprising urokinase and transmembrane domains in a variety of membrane proteins.

### Best Mode for Carrying Out the Invention

In a method for producing antibodies according to the invention, the expression vector to be inoculated to animals may be constructed as an expression vector having a fusion polynucleotide that consists of a polynucleotide encoding an antigenic protein and a polynucleotide encoding a transmembrane domain.

As for an antigenic protein, any one that can generate an antigen-antibody reaction in vivo may be used. The polynucleotide encoding an antigenic protein may be any one of genomic DNA, cDNA, synthetic DNA, etc., as far as it has an open reading frame (ORF). When the antigenic protein is an inherent secretory protein, it is used after removal of the signal sequence peptide originally possessed by the protein.

As for the transmembrane domain, any domain may be used as far as its N-terminal side is in the cell and the C-terminal side is out of the cell. For example, transmembrane domains of type II-membrane proteins or those of multispan-type membrane proteins may be used. The proteins that an antigenic protein is fused to the C-terminal side of these transmembrane domains take forms that the antigenic protein portion exists on the surface of the cell membrane. As for the transmembrane domain, for example, that of human type-II membrane protein HP10085 (SEQ ID NO: 2) may be used. In this case, the transmembrane domain to be fused with an antigenic protein is a polypeptide containing at least 1st methionine (Met) to 26th lysine (Lys) of SEQ ID NO: 2. The polynucleotide encoding the polypeptide contains at least the base sequence from 151st - 228th of SEQ ID NO: 1 (cDNA of human type-II membrane protein HP10085). This polynucleotide is linked to a polynucleotide encoding the above-mentioned antigenic protein, and the expression vector can be constructed using the fusion polynucleotide which expresses a fusion protein that an antigenic protein is fused to the C-terminal side of the transmembrane domain (polypeptide).

As for vectors expressing fusion proteins, any ones for eucaryotic cells may be used as far as they contain a promoter, a splicing region, a poly (A) addition site, etc., such as pKA1, pCDM8, pSVK3, pMSG, pSVL, pBK-CMV, pBK-RSV, EBV vectors, and the like. The two above-described polynucleotides are cloned to these vectors to make expression vectors that express respectively coded proteins as fusion proteins.

Non-human animals to which an expression vector is inoculated include mammals such as mouse, rat, rabbit, etc., and birds such as chicken, etc., which are generally used in producing antibodies. Inoculation of the expression vectors to animals may preferably be achieved using a gene gun technology and the like. When a gene gun technology is used, the expression vector is adsorbed on gold particles and emitted as a jet to the skin with gas pressure and the like for inoculation. The amount of the expression vector to be inoculated is variable depending on the species of animal and may preferably be in a range of 0.1 pg- 1 mg/animal. One-shot inoculation is acceptable, but it is desirable to carry out the inoculation twice or more at regular intervals in order to assure generation of the antibodies.

The generation of the antibody may be confirmed by collecting blood, separating serum, and examining the binding reaction with the antigenic protein. For example, a known method such as enzyme immunoassay (ELISA), Western blotting, immuno-precipitation, antibody staining, and the like may be used. After confirmation of the presence of the antibody in the serum by these methods, the serum may be used as a polyclonal antibody specimen as it is or may be purified by affinity column chromatography to yield IgG. Alternatively, the spleen may be taken out from the animal acquiring immunity and the monoclonal antibody can be produced in a conventional manner.

### Examples

The following examples serve to illustrate the invention in more detail and specifically but are not intended as a limitation thereof. In these examples, basic procedures for recombination of DNA and enzyme reactions are carried out according to the articles, "Molecular Cloning; A laboratory manual", Cold Spring Harbor Laboratory, 1989. Restriction enzymes and a variety of modified enzymes were obtained from Takara Shuzo Co., Ltd., unless otherwise stated. The compositions of buffer solutions in respective enzyme reactions and the reaction conditions were set according to the specification attached.

### (1) Construction of an Expression Vector for the Urokinase-Fusion Protein

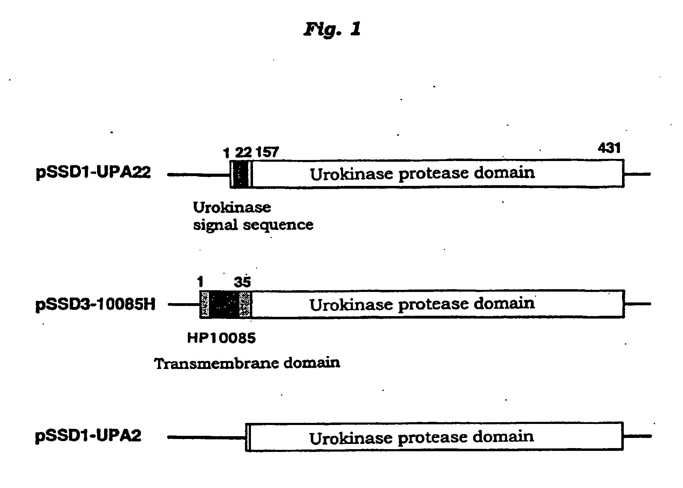

When urokinase is used as an antigenic protein, 3 kinds of expression vectors were used, that is, for secretion expression, for membrane form expression, and for intracellular expression. That is, the following vectors were respectively used: for secretion expression, pSSD1-UPA22 which expresses the signal sequence and protease domain of urokinase (Yokoyama-Kobayashi et al., Gene 163: 193-196, 1995); for membrane form expression, pSSD3-10085H which expresses a protein prepared by fusing a sequence from the N-terminal side to the 35th proline (Pro) of type II-membrane protein HP10085 (SEQ ID NO: 2) with the protease domain of urokinase (Yokoyama-Kobayashi et al., Gene 228: 161-167, 1999); for intracellular expression, pSSD1-UPA2 which expresses only the protease domain of urokinase by eliminating the signal sequence of urokinase (Yokoyama-Kobayashi et al., Gene 163: 193-196, 1995). Fig. 1 shows the structure of respective fusion gene portions. In every case, the structure is the same except the portion encoding fusion protein. That is, they have the early promoter of SV40, 16S mRNA splicing region of SV40 and poly (A) addition site of SV40.

### (2) Construction of an Expression Vector for the Fusion Protein with Nuclear Protein

As an antigenic protein, protein HP10496 localized in the nuclear spliceosome was used. cDNA clone pHP10496 encoding this protein was cloned from a human gastric cancer cDNA library (WO98/21328). This has the base sequence of SEQ ID NO: 3 as well as ORF which encodes protein HP10496 comprising the amino acid sequence of SEQ ID NO: 4. Using a 30mer sense primer (oligonucleotide of SEQ ID NO: 5) starting from a translation initiation codon to which a EcoRV recognition site was added and a 30 mer antisense primer (oligonucleotide of SEQ ID NO: 6) containing a stop codon to which an EcoRV recognition site was added, the translation region was amplified by PCR using pHP10496 as template. The PCR product was digested with EcoRV, and inserted into an EcoRV-NotI (blunt ends) site of a membrane-type of urokinase expression vector pSSD3-10085N to construct a fusion gene expression vector pHP10085N-10496.

In order to obtain an antigenic protein for antibody detection, an expression vector for expressing a protein fused with glutathione-S-transferase (GST) in Escherichia coli was constructed. Using a 28mer sense primer (oligonucleotide of SEQ ID NO: 7) starting from a translation initiation codon to which a EcoRI recognition site was added and a 32 mer antisense primer (oligonucleotide of SEQ ID NO: 8) containing a stop codon to which a SalI recognition site was added, the translation region was amplified by PCR using pHP10496 as template. The PCR product was digested with EcoRI and Sail and inserted into the EcoRI- Sail site of pGEX-5X-1 (Pharmacia Corp.). After confirmation of the sequence, a host Escherichia coli BL21 was transformed and incubated on an LB medium at 37°C for 5 hours. Then, IPTG was added at a final concentration of 0.4 mM, and the mixture was further incubated at 37°C for 2.5 hours. The cells were centrifuged, dissolved in a lysis buffer solution (50mM Tris-HCl (pH7.5), 1 mM EDTA, 1% Triton X-100, 0.2% SDS, 0.2 mM PMSF), frozen once at -80°C and thawed, and destroyed by sonication. After centrifugation at 1000 × g for 30 minutes, Glutathione Sepharose 4B was added to the supernatant, and the mixture was incubated at 4°C for 1 hour. The beads were washed well, and the fusion protein was eluted with an elution buffer solution (10 mM Tris-HCl, 50 mM glutathione). As a result, fusion protein GST-HP10496 having molecular weight of approximately 47 kDa was obtained.

### (3) Gene Immunization

Gene immunization was achieved with a gene gun (Helios Gene Gun System; Japan Bio-Rad Laboratories). Expression vector plasmid DNA was attached onto gold particles according to the protocol. DNA-coated gold particles corresponding to 2 µg of plasmid DNA were shot into the skin of inguinal region of 3 mice (BALB/c) per sample. The immunization was carried out twice a week for 4 weeks, and the blood was then collected.

### (4) Detection of the Antibody by ELISA

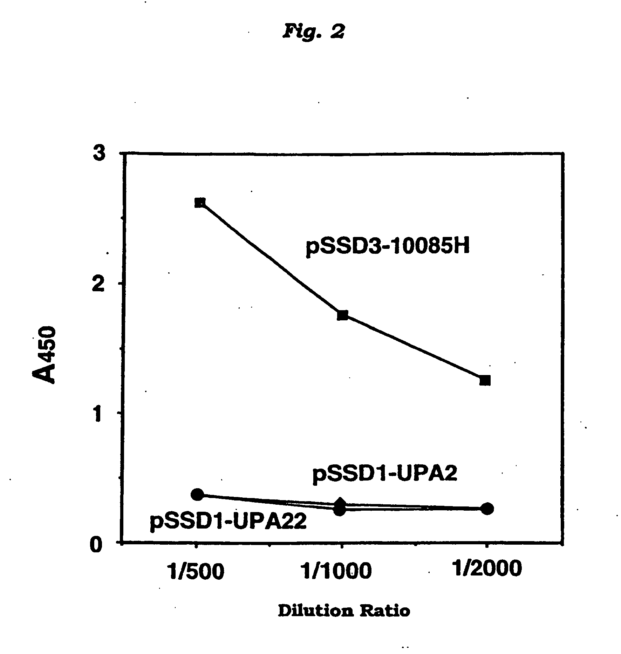

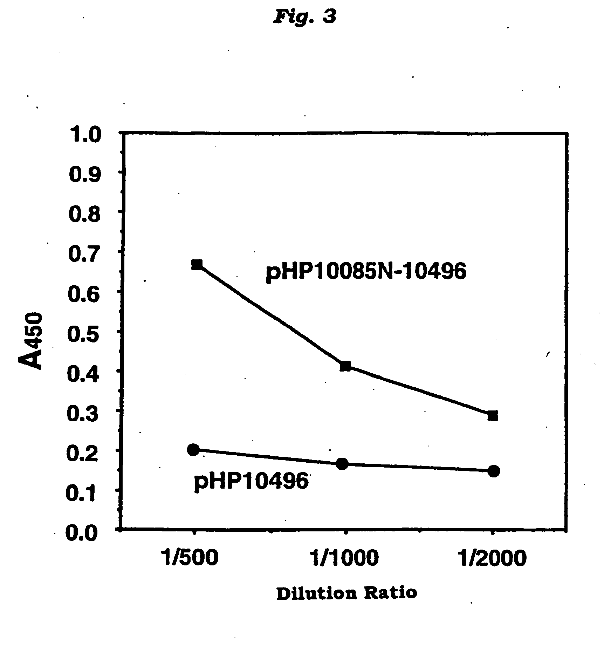

As an antigenic protein, commercially available urokinase (Wakamori Jun-yaku) or GST-HP10496 expressed in Escherichia coli was coated on a plate and it was used in ELISA. The serum obtained by gene immunization with urokinase or nuclear protein HP10496 as antigen was used to measure the titer of the antibodies. The results are shown in Figs. 2 and 3. In any cases, production of antibodies was recognized only in immunization with the vector expressing the proteins to which the N-terminal of HP10085 was fused.

### (5) Gene Immunization with Fusion Proteins Comprising the Transmembrane Domain of various species of Type II-Membrane Proteins and Urokinase

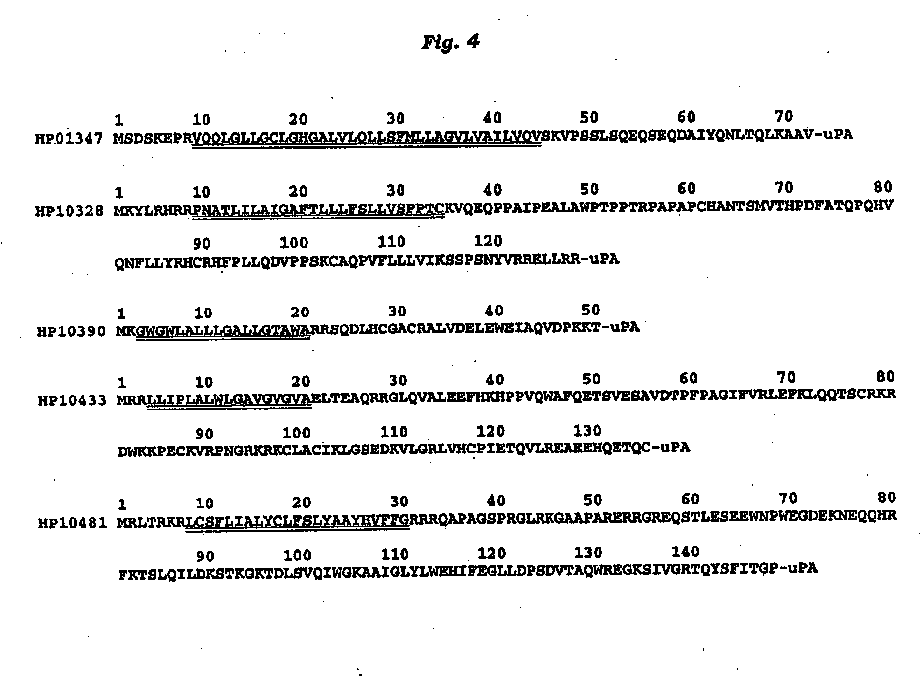

From a human full-length cDNA data bank, the following 5 species of type II-membrane protein cDNAs, HP01347 (SEQ ID NO: 9), HP10328 (SEQ ID NO: 10), HP10390 (SEQ ID NO: 11), HP10433 (SEQ ID NO: 12), and HP10481 (SEQ ID NO: 13) were identified, and the vectors expressing fusion proteins of urokinase with the respective transmembrane domains were constructed (Yokoyama-Kobayashi et al., Gene 228: 161-167, 1999). Fig. 4 shows the respective N-terminal amino acid sequences.

The gene immunization was carried out with these expression vectors according to the method as described in the above item (3), and the antibodies to urokinase were detected according to the method as described in the above item (4). In respective cases, production of antibodies was recognized in the same as in HP10085.

### Industrial Applicability

According to the present invention, an antibody against an antigenic protein, which it was difficult to produce in the so far known gene immunization, can be produced. The resulting an antibody is useful as drugs, diagnostic agents, and reagents for the research.

## Claims

1. A method for producing an antibody against an antigenic protein, which method comprises (i) inoculating a non-human animal with an expression vector expressing a fusion protein, wherein said fusion protein is an antigenic protein fused with the C-terminal side of a transmembrane domain of which the N-terminal side is located in the cell and the C-terminal side is out of the cell, (ii) isolating said antibody against said antigenic protein from the non-human animal and (iii) purifying the antibody.

2. The method of claim 1, wherein the transmembrane domain is from a type II-membrane protein.

3. The method of claim 1, wherein the transmembrane domain is from Human type II membrane protein HP 10085.

4. The method of any one of claims 1 to 3, wherein the transmembrane domain is a polypeptide having at least the amino acid sequence from 1st to 26th of SEQ ID NO. 2.

5. Use of a fusion polynucleotide which encodes a fusion protein in which an antigenic protein is fused with the C-terminal side of a transmembrane domain of which the N-terminal side is located in the cell and the C-terminal side is out of the cell or of an expression vector containing said fusion polynucleotide or of a eukaryotic cell containing said expression vector for the production of an antibody against the antigenic protein.

6. The use of claim 5, wherein the transmembrane domain is from a type II-membrane protein or a multi span-type membrane protein.

7. The use of claim 5, wherein the transmembrane domain is from Human type II membrane protein HP 10085.

8. The use of claim 6, wherein the encoded transmembrane domain has at least the amino acid sequence from 1st to 26th of SEQ ID NO. 2.

9. A non-human animal inoculated with an expression vector as defined in claim 5.

## Patentansprüche

1. Ein Verfahren zur Herstellung eines Antikörpers gegen ein antigenes Protein, wobei das Verfahren umfasst (i) das Beimpfen eines nichtmenschlichen Tieres mit einem Expressionsvektor, der ein Fusionsprotein exprimiert, wobei das Fusionsprotein ein antigenes Protein ist, fusioniert mit der C-terminalen Seite einer Transmembrandomäne, deren N-terminale Seite sich in der Zelle befindet und deren C-terminale Seite sich außerhalb der Zelle befindet, (ii) das Isolieren des Antikörpers gegen das antigene Protein von dem nichtmenschlichen Tier und (iii) das Reinigen des Antikörpers.

2. Das Verfahren gemäß Anspruch 1, wobei die Transmembrandomäne von einem Typ-II-Membranprotein stammt.

3. Das Verfahren gemäß Anspruch 1, wobei die Transmembrandomäne von menschlichem Typ-II-Membranprotein HP 10085 stammt.

4. Das Verfahren gemäß einem der Ansprüche 1 bis 3, wobei die Transmembrandomäne ein Polypeptid mit wenigstens der Aminosäuresequenz von 1. bis 26. von SEQ ID NR. 2 ist.

5. Verwendung eines Fusionspolynukleotids, welches für ein Fusionsprotein kodiert, bei dem ein antigenes Protein mit der C-terminalen Seite einer Transmembrandomäne, deren N-terminale Seite sich in der Zelle befindet und deren C-terminale Seite sich außerhalb der Zelle befindet, fusioniert ist, oder eines Expressionsvektors, der das Fusionspolynukleotid enthält, oder einer eukaryotischen Zelle, die den Expressionsvektor enthält, zur Herstellung eines Antikörpers gegen das antigene Protein.

6. Die Verwendung gemäß Anspruch 5, wobei die Transmembrandomäne von einem Typ-II-Membranprotein oder einem Multi-Span-Typ-Membranprotein stammt.

7. Die Verwendung gemäß Anspruch 5, wobei die Transmembrandomäne von einem menschlichen Typ-II-Membranprotein HP 10085 stammt.

8. Die Verwendung gemäß Anspruch 6, wobei die kodierte Transmembrandomäne wenigstens die Aminosäuresequenz von 1. bis 26. von SEQ ID NR. 2 besitzt.

9. Ein nichtmenschliches Tier, das mit einem wie in Anspruch 5 definierten Expressionsvektor beimpft ist.

## Revendications

1. Procédé de production d'un anticorps dirigé contre une protéine antigénique, lequel procédé comprend :
(i) l'inoculation à un animal non humain d'un vecteur d'expression exprimant une protéine de fusion, dans lequel ladite protéine de fusion est une protéine antigénique fusionnée avec la partie C-terminale d'un domaine transmembranaire dont le côté N-terminal est situé dans la cellule et le côté C-terminal est situé à l'extérieur de la cellule, (ii) l'isolement dudit anticorps dirigé contre ladite protéine antigénique à partir de l'animal non humain, et (iii) la purification de l'anticorps.

2. Procédé selon la revendication 1, dans lequel le domaine transmembranaire provient d'une protéine membranaire de type II.

3. Procédé selon la revendication 1, dans lequel le domaine transmembranaire provient de la protéine membranaire humaine de type II HP 10085.

4. Procédé selon l'une quelconque des revendications 1 à 3, dans lequel le domaine transmembranaire est un polypeptide ayant au moins la séquence d'acides aminés s'étendant de la position 1 à la position 26 de SEQ ID n° 2.

5. Utilisation d'un polynucléotide de fusion qui code pour une protéine de fusion, dans laquelle une protéine antigénique est fusionnée avec le côté C-terminal d'un domaine transmembranaire, dont le côté N-terminal est situé dans la cellule et le côté C-terminal est situé à l'extérieur de la cellule, ou d'un vecteur d'expression contenant ledit polynucléotide de fusion, ou d'une cellule eucaryote contenant ledit vecteur d'expression, pour la production d'un anticorps dirigé contre la protéine antigénique.

6. Utilisation selon la revendication 5, dans laquelle le domaine transmembranaire provient d'une protéine membranaire de type II ou d'une protéine membranaire présentant plusieurs passages transmembranaires.

7. Utilisation selon la revendication 5, dans laquelle le domaine transmembranaire provient de la protéine membranaire humaine de type II HP 10085.

8. Utilisation selon la revendication 6, dans laquelle le domaine transmembranaire codé a au moins la séquence d'acides aminés s'étendant de la position 1 à la position 26 de SEQ ID n° 2.

9. Animal non humain inoculé avec un vecteur d'expression, tel que défini selon la revendication 5.
